Europäisches Patentamt

European Patent Office

Office européen des brevets

(11) Veröffentlichungsnummer: **0 174 531 B1**

# EUROPÄISCHE PATENTSCHRIFT

(45) Veröffentlichungstag der Patentschrift: **20.05.92**

(21) Anmeldenummer: **85110540.3**

(22) Anmeldetag: **22.08.85**

(51) Int. Cl.⁵: **A61F 2/38**

(54) **Endoprothese eines Gelenks.**

(30) Priorität: **29.08.84 DE 3431645**

(43) Veröffentlichungstag der Anmeldung:
**19.03.86 Patentblatt 86/12**

(45) Bekanntmachung des Hinweises auf die
Patenterteilung:
**20.05.92 Patentblatt 92/21**

(84) Benannte Vertragsstaaten:
**CH FR GB LI NL SE**

(56) Entgegenhaltungen:
**DE-A- 3 119 841**
**DE-B- 2 549 318**
**US-A- 4 224 697**
**US-A- 4 262 368**
**US-A- 4 298 992**

(73) Patentinhaber: **GMT Gesellschaft für medizinische Technik mbH**
**Holstenstrasse 2**
**W-2000 Hamburg 50(DE)**

Patentinhaber: **Waldemar Link GmbH & Co**
**Barkhausenweg 10**
**W-2000 Hamburg 63(DE)**

(72) Erfinder: **Engelbrecht, Eckart, Dr.**
**Endo-Klinik Hamburg Holstenstrasse 2**
**W-2000 Hamburg 50(DE)**
Erfinder: **Nieder, Elmar, Dr.**
**Endo-Klinik Hamburg Holstenstrasse 2**
**W-2000 Hamburg 50(DE)**
Erfinder: **Keller, Arnold**
**An der Naherfurth 5**
**W-2061 Kayhude(DE)**

(74) Vertreter: **Heldt, Gert, Dr. Dipl.-Ing.**
**Neuer Wall 59 III**
**W-2000 Hamburg 36(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 174 531 B1

**Beschreibung**

Die Erfindung betrifft eine Endoprothese eines Gelenkes bestehend aus zwei miteinander unverbundenen Komponenten, die in Richtung des zwischen ihnen bestehenden Kraftflusses gegeneinander verschieblich gelagert sind.

Diese Prothese hat sich insbesondere in Form einer Kniegelenkprothese zum Ersatz eines zerstörten Gelenkes gut bewährt. Die implantierten Kniegelenke ermöglichen ihren Trägern Bewegungsmöglichkeiten, die denen natürlicher Kniegelenke weitgehend angenähert sind. Hierfür hat sich als besonders günstig die Verschieblichkeit der Komponenten gegeneinander herausgestellt. Die Verschiebungen der Komponenten in voneinander abgewandte Richtungen werden begrenzt durch den die Endoprothese umgebenden Weichteilapparat, sofern dieser weitgehend seine Funktion erfüllen kann.

Aus der US-A-4 224 697 ist eine Kniegelenkendoprothese bekannt, die ein zentrales, den Hubweg begrenzendes Element aufweist. Dadurch entstehen im Falle eines oberen oder unteren Anschlages aufgrund der Masseträgheit sehr hohe Kraftdichten je Flächeneinheit. Diese können insbesondere bei langandauernden und wiederkehrenden Rotationsbewegungen zu Materialverformungen führen, die die ungehinderte Rotationsfähigkeit der Prothese beeinträchtigen.

Aufgabe der vorliegenden Erfindung ist es daher, eine Kniegelenkendoprothese der einleitend genannten Art so auszubilden, daß sie auch bei weitgehend zerstörtem Weichteilapparat eine langandauernde zerstörungsfreie Benutzung gewährleistet.

Diese Aufgabe wird erfindungsgemäß dadurch gelöst, daß zwischen den Komponenten mindestens ein das Maß der Verschieblichkeit der Komponenten gegeneinander begrenzender Anschlag angeordnet ist, der als ein das Führungsstück radial mindestens teilweise umgebender Kragen ausgebildet ist, der eine an der anderen Komponente vorgesehene Anschlagsleiste hintergreift.

Die Kombination aus Kragen und Anschlagleiste ermöglicht die Ausbildung eines Hubbereiches, der eine freie Längsbewegung der ersten Komponente entlang der Gelenklängsachse ermöglicht, die eine weiche Abbremsung einer Rotationsbewegung der ersten Gelenkkomponente um die Längsachse durch eine Ergänzung der Rotationsbewegung durch eine Längsbewegung zuläßt. Die durch eine Führung der ersten Komponente entlang von Auflaufflächen hervorgerufene Ergänzung der Bewegung durch die Längskomponente erzeugt bei mindestens teilweise funktionsfähigem Weichteilapparat die Längsbewegungder ersten Gelenkkomponente und damit auch deren Rotationsbewegung in gegengerichtete Kräfte. Ein harter, die Rotationsbewegung begrenzender Anschlag, dessen häufiges Wirksamwerden zu einer Lockerung der Prothese führen würde, wird hierdurch vermieden. Durch die Begrenzung des Hubbereiches wird auch bei einem bereits zum Operationszeitpunkt beschädigten Weichteilapparat oder einer erst nach der Operation eingetretenen Schwächung des Weichteilapparates eine anatomisch zulässige Führung der Endoprothese gewährleistet. Vor Erreichen der Grenze des Zulässigkeitsbereiches wird die Längsbewegung der ersten Gelenkkomponente entlang der Gelenklängsachse durch einen Anschlag des Kragens an die Anschlagleiste begrenzt.

Gemäß einer bevorzugten Ausführungsform der Erfindung ist die Prothese als Kniegelenkprothese mit einer femoralen und einer tibialen Komponente ausgebildet. Wegen der in einem Kniegelenk ablaufenden Bewegungsabläufe sollte gerade bei diesem Gelenk die Verschieblichkeit der Komponenten gegeneinander durch einen Anschlag begrenzt werden, um eine langandauernde Funktionsfähigkeit der Prothese zu gewährleisten unter weitgehender Schonung der Knochen und des Weichteilapparates.

Weitere Einzelheiten der Erfindung ergeben sich aus der nachfolgenden ausführlichen Beschreibung und den beigefügten Zeichnungen, in denen eine bevorzugte Ausführungsform der Erfindung beispielsweise veranschaulicht ist.

In den Zeichnungen zeigen:

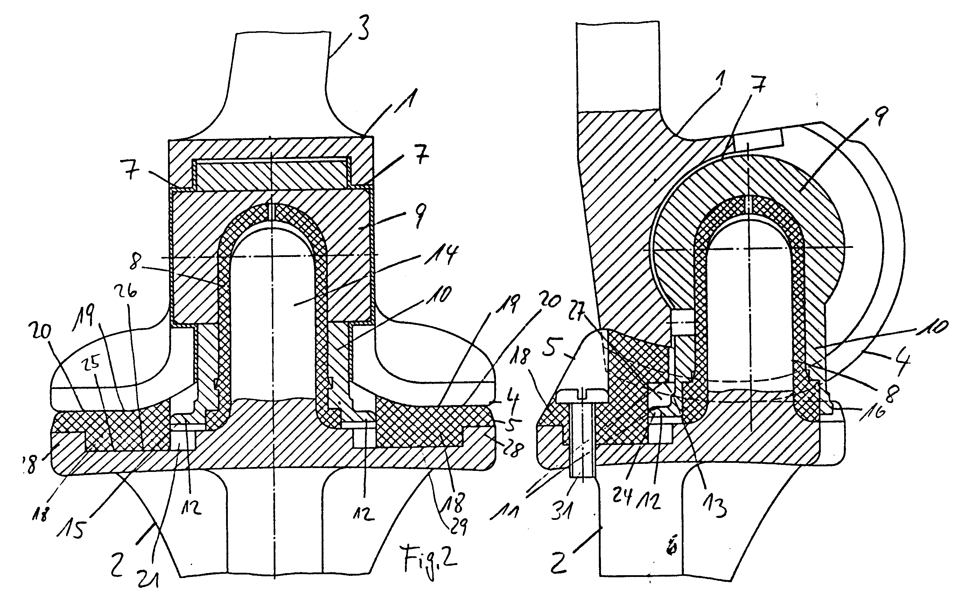

Fig. 1 Eine Seitenansicht einer Endoprothese in teilweise geschnittener Darstellung

Fig. 2 eine Vorderansicht einer Endoprothese in teilweise geschnittener Darstellung

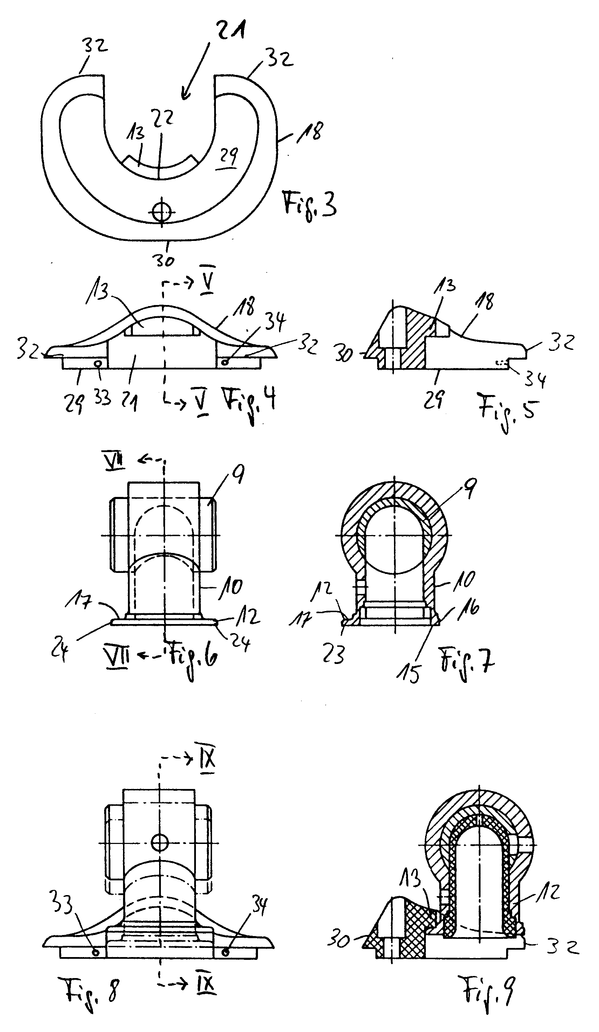

Fig. 3 eine Druntersicht unter eine Lagerplatte

Fig. 4 eine Rückansicht einer Lagerplatte

Fig. 5 einen Längsschnitt durch eine Lagerplatte entlang der Linie V-V in Fig. 4

Fig. 6 eine Vorderansicht eines als Scharnierbolzen ausgebildeten Führungsstückes

Fig. 7 einen Schnitt entlang der Linie VII-VII in Fig. 6

Fig. 8 eine Rückansicht eines mit einer Lagerplatte zusammengesetzten Führungsstückes und

Fig. 9 einen Schnitt entlang der Linie IX-IX in Fig. 8.

Die Endoprothese eines Gelenkes wird am Beispiel einer Kniegelenkendoprothese erläutert. Die Endoprothese eines Kniegelenkes besteht im wesentlichen aus zwei Komponenten 1, 2, von denen eine als femorale Komponente 1 und die andere als tibiale Komponente 2 ausgebildet ist. Bei der femoralen Komponenten 1 schließt sich an einen Stiel 3 der in einen Oberschenkelknochen eines Patienten eingesetzt wird, eine femorale Lagerhälfte 4 an. Diese Lagerhälfte 4 stützt sich auf einer tibialen Lagerhälfte 5 ab, die über einen Stiel 6 an einem Unterschenkelknochen des Patienten befestigt wird. Zwischen den Komponenten 1, 2 ist ein Beugebewegungen um deren Querachsen gegeneinander zulassendes erstes Lager 7 und ein Rotationsbewegungen um die Längsachsen der Komponenten 1, 2 gegeneinander zulassendes zweites Lager 8 angeordnet. Das erste Lager 7 besteht aus einem Schwenklager. Es ist als ein an der einen Komponente 1 befestigtes Scharnier ausgebildet, das ein als Scharnierbolzen 9 ausgebildetes Führungsstück 1o aufweist, das rotationsbeweglich und schiebebeweglich auf der anderen Komponente 2 gelagert ist. Zwischen den Komponenten 1, 2 ist ein Anschlag 11 vorgesehen. Dieser ist als ein das Führungsstück 1o radial zumindest teilweise umbender Kragen 12 ausgebildet, der eine an der anderen Komponente 2 vorgesehene Anschlagleiste 13 hintergreift. Der Kragen 12 ist an der femoralen Komponente 1 und die Anschlagleiste 13 an der tibialen Komponente 2 vorgesehen. Das das erste Lager 7 ausbildende Scharnier ist in der femoralen Komponente 1 befestigt. In das Führungsstück 1o ragt ein dessen Rotationsbewegungen und Schiebebewegungen zulassender Zapfen 14 hinein, der fest mit der tibialen Komponente 2 verbunden ist. Der Kragen 12 ist an einem der tibialen Komponente 2 zugekehrten Ende 15 des Führungsstückes 1o angeordnet. Der Kragen 12 überragt das Führungsstück 1o mindestens teilweise in Richtungen, die dem Zapfen 14 abgewandt sind. Er weist die Form eines Ringflansches auf, der an seiner der tibialen Komponente 2 abgewandten Seite 16 eine Abstützfläche 17 aufweist, auf der sich die Anschlagleiste 13 bei Erreichen einer vorgewählten Endstellung abstützt, in der die Komponenten 1, 2 am weitesten auseinander geschoben sind. Der Kragen 12 ist in einem hinteren Bereich des Führungsstückes 1o abgeflacht, in dem sich die beiden Komponenten 1, 2 bei Ausführung der Beugebewegungen am weitesten annähern. Die Anschlagleiste 13 ist an der tibialen Lagerhälfte 5 vorgesehen, die die tibiale Komponente 2 in Richtung auf die femorale Komponente 1 begrenzt. Die tibiale Lagerhälfte 5 besteht aus einer im wesentlichen hufeisenförmigen Lagerplatte 18, die den Zapfen 14 teilweise umgibt. Sie erstreckt sich im wesentlichen quer zur Längsrichtung der tibialen Komponente 2. Auf ihrer der femoralen Komponente 1 zugekehrten Oberseite 19 ist sie mit einer Gleitfläche 2o versehen, auf der sich die femorale Lagerhälfte 4, 5 abstützt. Die Lagerplatte 18 ist mit einer im wesentlichen U-förmigen Ausnehmung 21 versehen, die sie im wesentlichen in Längsrichtung der tibialen Komponente 2 durchdringt. Die Ausnehmung 21 weist einen vorderen abgerundeten Bereich 22 auf, der einem vorderen Bereich des Kragens 12 zugewandt ist, der die Anschlagleiste 13 hintergreift. Die Ausnehmung 21 ist in ihrem abgerundeten Bereich 22 einem ihm zugewandten Teil 24 des Kragens 12 angepaßt. Die Anschlagleiste 13 ragt in den abgerundeten Bereich 22 der Ausnehmung 21 hinein und begrenzt den abgerundeten Bereich 22 in Richtung auf die femorale Komponente 1. Die Anschlagleiste 13 ist gebogen und verläuft den abgerundeten Bereich 22 im wesentlichen konzentrisch. Sie hält einen Abstand zu einer Oberseite 25 eines die Lagerplatte 18 tragenden Auflagers 26 der tibialen Komponente 2 ein. Zwischen der Anschlagleiste 13 und der Oberseite 25 ist ein Freiraum 27 vorgesehen, der die Höhe des Abstandes zwischen der Anschlagleiste 13 und der Oberseite 25 aufweist. Der Kragen 12 ist verschieblich und drehbeweglich in dem Freiraum 27 gelagert und weist gegenüber der Ausnehmung 21 ein Spiel auf. Die Lagerplatte 18 ist an dem Auflager 26 befestigt. Dieses ist an seiner Oberseite 25 von einem Wulstrand 28 umgeben, an den die Lagerplatte 18 mit ihrer dem Auflager 26 zugekehrten Unterseite 29 angepaßt ist. Die Lagerplatte 18 ist der Oberseite 25 formschlüssig angepaßt. Sie ist in ihrem vorderen Bereich 3o mit einer Schraube 31 an dem Auflager 26 befestigt, die mindestens teilweise in die Lagerplatte 18 versenkt ist. Die Lagerplatte 18 ist in ihren dem vorderen Bereich 3o abgewandten hinteren Bereichen 32 mit je einem Sackloch 33, 34 versehen, die einander parallel und in einer von der Lagerplatte 18 aufgespannten Ebene verlaufen. In die Sacklöcher 33, 34 ragen Sicherheitsstifte hinein, die den Wulstrand 28 in Richtung auf die Sacklöcher 33, 34 überragen.

Die Endoprothese eines Kniegelenkes kann wie folgt eingesetzt werden:

Die femorale Komponente ist bereits in der Weise vormontiert, daß das Führungsstück 1o das die Beugebewegungen zulassende erste Lager 7 ausbildet. Nachdem die beiden Komponenten 1, 2 jeweils mit den ihnen zugekehrten Oberschenkelbeziehungsweise Unterschenkelknochen verbunden sind, wird der Zapfen 14 in das Führungsstück 1o eingeschoben, so daß der Zapfen 14 mit dem Führungsstück 1o das Rotationsbewegungen und Schiebebewegungen der Komponenten 1, 2 gegeneinander zulassende zweite Lager 8 ausbildet. Zwischen die Komponenten 1, 2 wird die Lagerplatte 18 in der Weise eingeschoben, daß die Sacklöcher 33, 34 die an dem Wulstrand 28 vorgesehenen Sicherungsstifte aufnehmen und der Kragen 12 in den Freiraum 21 hineinragt. Danach wird die Lagerplatte 18 auf das Auflager 26 abgesenkt, so daß sie mit dem Auflager 26 und dem Wulstrand 28 eine formschlüssige Verbindung aufweist. Die Lagerplatte 18 wird mit Hilfe der Schraube 31 an dem Auflager 26 festgeschraubt.

Nunmehr ist die Endoprothese eines Kniegelenkes fertig montiert. Ein mit dieser Prothese versehenes Bein kann weitgehend wie ein mit einem natürlichen Knie versehenes Bein bewegt werden. Dies wird dadurch ermöglicht, daß die Komponenten 1, 2 gegeneinander verschwenkt, verdreht und verschoben werden können. Auch wenn der die Kniegelenkendoprothese umgebende Weichteilapparat nicht mehr in der Lage ist, ein Auseinanderschieben der Komponenten 1, 2 zu begrenzen oder zu verhindern, kann die Endoprothese eingesetzt werden, da der zwischen dem Kragen 12 und der Anschlagleiste 13 ausgebildete Anschlag 11 ein Auseinanderschieben der Komponenten 1, 2 über ein vorgewähltes Maß hinaus verhindert.

## Patentansprüche

1. Endoprothese eines Gelenkes bestehend aus zwei miteinander unverbundenen Komponenten (1, 2), die in Richtung des zwischen ihnen bestehenden Kraftflusses gegeneinander verschieblich gelagert sind, dadurch gekennzeichnet, daß zwischen den Komponenten (1, 2) mindestens ein Maß der Verschieblichkeit der Komponenten (1, 2) gegeneinander begrenzender Anschlag (11) angeordnet ist, der als ein das Führungsstück (10) radial mindestens teilweise umgebender Kragen (12) ausgebildet ist, der eine an der anderen Komponente (2) vorgesehene Anschlagsleiste (13) hintergreift.

2. Endoprothese nach Anspruch 1, dadurch gekennzeichnet, daß sie als Kniegelenkendoprothese mit einer femoralen und tibialen Komponente (1, 2) ausgebildet ist.

3. Endoprothese nach Anspruch 1 und 2, dadurch gekennzeichnet, daß zwischen den Komponenten (1, 2) mindestens je ein Beugebewegungen um deren Querachsen und Rotationsbewegungen um deren Längsachsen jeweils gegeneinander zulassendes Lager (7, 8) angeordnet ist.

4. Endoprothese nach Anspruch 1 bis 3, dadurch gekennzeichnet, daß ein die Beugebewegungen zulassendes Schwenklager als ein an der einen Komponente (1) befestigtes Scharnier ausgebildet ist, das ein als Scharnierbolzen (9) ausgebildetes Führungsstück (10) aufweist, das rotationsbeweglich und schiebebeweglich auf der anderen Komponente (2) gelagert ist.

5. Endoprothese nach Anspruch 2 bis 4, dadurch gekennzeichnet, daß der Kragen (12) an der femoralen Komponente (1) und die Anschlagleiste (13) an der tibialen Komponente (2) vorgesehen ist.

6. Endoprothese nach Anspruch 4 und 5, dadurch gekennzeichnet, daß das Scharnier an der femoralen Komponente (1) befestigt ist und in das Führungsstück (10) ein dessen Rotationsbewegungen und Schiebebewegungen zulassender Zapfen (14) hineinragt, der fest mit der tibialen Komponente (2) verbunden ist und der Kragen(12) an einem der tibialen Komponente (2) zugekehrten Ende (15) des Führungsstükkes (10) angeordnet ist.

7. Endoprothese nach Anspruch 6, dadurch gekennzeichnet, daß der Kragen (12) das Führungsstück (10) in Richtungen mindestens teilweise überragt, die dem Zapfen (14) abgewandt sind.

8. Endoprothese nach Anspruch 7, dadurch gekennzeichnet, daß der Kragen (12) das Führungsstück (10) in Form eines Ringflansches überragt, der an seiner der tibialen Komponente (2) abgewandten Seite eine Abstützfläche (17) aufweist, auf der sich die Anschlagleiste (13) bei Erreichen einer vorgewählten Endstellung abstützt, in der die Komponenten (1, 2) am weitesten auseinandergeschoben sind.

9. Endoprothese nach Anspruch 3 bis 8, dadurch gekennzeichnet, daß der Kragen (12) in einem hinteren Bereich (16) des Führungsstückes (10) abgeflacht ist, in dem sich die beiden Komponenten (1, 2) bei Ausführung der Beugebewegungen am weitesten annähern.

10. Endoprothese nach Anspruch 2 bis 9, dadurch gekennzeichnet, daß die Anschlagleiste (13) an einer tibialen Lagerhälfte (5) vorgesehen ist, die die tibiale Komponente (2) in Richtung auf die femorale Komponente (1) begrenzt.

11. Endoprothese nach Anspruch 10, dadurch gekennzeichnet, daß die tibiale Lagerhälfte (5) aus einer im wesentlichen hufeinsenförmigen Lagerplatte (18) besteht, die den Zapfen (14) teilweise umgibt, die sich im wesentlichen quer zur Längsrichtung der tibialen Komponente (2) erstreckt und die an ihrer der femoralen Komponente (1) zugekehrten Oberseite (19) mit Gleitflächen (20) versehen ist, auf der sich eine femorale Lägerhälfte (4) abstützt.

12. Endoprothese nach Anspruch 11, dadurch gekennzeichnet, daß die Lagerplatte (18) eine im wesentlichen U-förmige Ausnehmung (21) aufweist, die sie im wesentlichen in Längsrichtung der tibialen Komponente (2) durchdringt und die einen vorderen abgerundeten Bereich (22) aufweist, der einem vorderen Bereich (23) des Kragens (12) zugewandt ist, der die Anschlagleiste (13) hintergreift.

13. Endoprothese nach Anspruch 12, dadurch gekennzeichnet, daß die Ausnehmung (21) in ihrem abgerundeten Bereich (22) einen ihm zugewandten Teil (24) des Kragens (12) angepaßt ist.

14. Endoprothese nach Anspruch 12 und 13, dadurch gekennzeichnet, daß die Anschlagleiste (13) in den abgerundeten Bereich (22) der Ausnehmung (21) in Richtung auf den Zapfen (12) hineinragt.

15. Endoprothese nach Anspruch 12 bis 14, dadurch gekennzeichnet, daß die Anschlagleiste (13) den abgerundeten Bereich (22) in Richtung auf die femorale Komponente (1) begrenzt.

16. Endoprothese nach Anspruch 12 bis 15, dadurch gekennzeichnet, daß die Anschlagleiste (13) gebogen ist und zu dem abgerundeten Bereich (22) im wesentlichen konzentrisch verläuft.

17. Endoprothese nach Anspruch 11 bis 16, dadurch gekennzeichnet, daß die Anschlagleiste (13) einen Abstand zu einer Oberseite (25) eines die Lagerplatte (18) tragenden Auflagers (26) der tibialen Komponente (2) einhält.

18. Endoprothese nach Anspruch 17, dadurach gekennzeichnet, daß zwischen der Anschlagleiste (13) und der Oberseite (25) ein Freiraum (27) vorgesehen ist, der die Höhe des Abstandes zwischen der Anschlagleiste (13) und der Oberseite (25) aufweist und der Kragen (12) verschieblich und drehbeweglich in dem Freiraum (27) gelagert ist.

19. Endoprothese nach Anspruch 12 bis 18, dadurch gekennzeichnet, daß der Kragen 612) gegenüber der Ausnehmung (21) ein Spiel aufweist.

20. Endoprothese nach Anspruch 17 bis 19, dadurch gekennzeichnet, daß die Lagerplatte (18) an dem Auflager (26) beafestigt ist.

21. Endoprothese nach Anspruch 17 bis 20, dadurch gekennzeichnet, daß das Auflager (26) an seiner Oberseite (25) von einem Wulstrand (28) umgeben ist, an dem die Lagerplatte (18) mit ihrer dem Auflager (26) zugewandten Unterseite (29) angepaßt ist.

22. Endoprothese nach Anspruch 17 bis 21, dadurch gekennzeichnet, daß die Lagerplatte (18) der Oberseite (25) formschlüssig angepaßt ist.

23. Endoprothese nach Anspruch 20 bis 22, dadurch gekennzeichnet, daß die Lagerplatte (18) in ihrem vorderen Bereich (30) mit einer Schraube (31) an dem Auflager (26) befestigt ist, die mindestens teilweise in der Lagerplatte (18) versenkt ist.

24. Endoprothese nach Anspruch 20 bis 23, dadurch gekennzeichnet, daß die Lagerplatte (18) in ihren dem vorderen Bereich (30) abgewandten Bereichen (32) mindestens je ein sackloch (33, 34) aufweist, die einander parallel und in einer von der Lagerplatte (18) aufgespannten Ebene verlaufen und in die Sicherungsstifte hineinragen, die den Wulstrand (28) in Richtuöng auf die Sacklöcher (33, 34) überragen.

**Claims**

1. An endoprosthesis of a joint comprising two components (1, 2) which are unconnected to each other and which are mounted displaceably relative to each other in the direction of the flow of force between them, characterised in that arranged between the components (1, 2) is at least one abutment (11) which limits the degree of displaceability of the components (1, 2) relative to each other and which is in the form of a collar (12) which at least partially radially surrounds the guide portion (10), the collar engaging behind an abutment bar (13) provided on the other component (2).

2. An endoprosthesis according to claim 1 characterised in that it is in the form of a knee joint endoprosthesis with femoral and tibial components (1, 2).

3. An endoprosthesis according to claim 1 and claim 2 characterised in that disposed between the components (1, 2) is at least one respective mounting (7, 8) which respectively permits bending movements about their transverse axes and rotational movements about their longitudinal axes relative to each other.

4. An endoprosthesis according to claims 1 to 3 characterised in that a pivot mounting which permits the bending movements is in the form of a hinge which is fixed to the one component (1) and which has a guide portion (10) in the form of a hinge pin (9), the guide portion being rotationally movably and slidably mounted on the other component (2).

5. An endoprosthesis according to claims 2 to 4 characterised in that the collar (12) is provided on the femoral component (1) and the abutment bar (13) is provided on the tibial component (2).

6. An endoprosthesis according to claim 4 and claim 5 characterised in that the hinge is fixed to the femoral component (1) and projecting into the guide portion (10) is a pin portion (14) which permits the rotational movements and sliding movements thereof and which is fixedly connected to the tibial component (2) and the collar (12) is arranged at an end (15) of the guide portion (10), which is towards the tibial component (2).

7. An endoprosthesis according to claim 6 characterised in that the collar (12) at least partially projects beyond the guide portion (10) in directions which are remote from the pin portion (14).

8. An endoprosthesis according to claim 7 characterised in that the collar (12) projects beyond the guide portion (10) in the form of an annular flange which, at its side remote from the tibial component (2), has a support surface (17) on which the abutment bar (13) bears when a preselected limit position in which the components (1, 2) are pushed furthest apart is reached.

9. An endoprosthesis according to claims 3 to 8 characterised in that the collar (12) is flattened in a rearward region (16) of the guide portion (10), in which the two components (1, 2) come closest together when performing the bending movements.

10. An endoprosthesis according to claims 2 to 9 characterised in that the abutment bar (13) is provided on a tibial mounting half (5) which limits the tibial component (2) in a direction towards the femoral component (1).

11. An endoprosthesis according to claim 10 characterised in that the tibial mounting half (5) comprises a substantially horseshoe-shaped mounting plate (18) which partially surrounds the pin portion (14) and which extends substantially transversely to the longitudinal direction of the tibial component (2) and which, at its top side (19) towards the femoral component (1), is provided with sliding surfaces (20) on which a femoral mounting half (4) is supported.

12. An endoprosthesis according to claim 11 characterised in that the mounting plate (18) has a substantially U-shaped recess (21) which passes therethrough substantially in the longitudinal direction of the tibial component (2) and which has a front rounded-off region (22) which is towards a front region (23) of the collar (12) which engages behind the abutment bar (13).

13. An endoprosthesis according to claim 12 characterised in that in its rounded-off region (22) the recess (21) is adapted to a portion (24), which is towards it, of the collar (12).

14. An endoprosthesis according to claim 12 and claim 13 characterised in that the abutment bar (13) projects into the rounded-off region (22) of the recess (21) in a direction towards the pin portion (14).

15. An endoprosthesis according to claims 12 to 14 characterised in that the abutment bar (13) limits the rounded-off region (22) in a direction towards the femoral component (1).

16. An endoprosthesis according to claims 12 to 15 characterised in that the abutment bar (13) is curved and extends substantially concentrically with respect to the rounded-off region (22).

17. An endoprosthesis according to claims 11 to 16 characterised in that the abutment bar (13) maintains a spacing relative to a top side (25) of a support (26), which carries the mounting plate (18), of the tibial component (2).

18. An endoprosthesis according to claim 17 characterised in that provided between the abutment bar (13) and the top side (25) is a free space (27) which is of a height corresponding to the spacing between the abutment bar (13) and the top side (25) and the collar (12) is mounted slidably and rotationally movably in the free space (27).

**19.** An endoprosthesis according to claims 12 to 18 characterised in that the collar (12) has a clearance relative to the recess (21).

**20.** An endoprosthesis according to claims 17 to 19 characterised in that the mounting plate (18) is feed to the support (26).

**21.** An endoprosthesis according to claims 17 to 20 characterised in that at its top side (25) the support (26) is surrounded by a rim portion (28) to which the mounting plate (18) is adapted with its underside (29) which is towards the support (26).

**22.** An endoprosthesis according to claims 17 to 21 characterised in that the mounting plate (18) is form-lockingly adapted to the top side (25).

**23.** An endoprosthesis according to claims 20 to 22 characterised in that the mounting plate (18) is fined in its front region (30) to the support (26) by a screw (31) which is at least partially sunk in the mounting plate (18).

**24.** An endoprosthesis according to claims 20 to 23 characterised in that in each of its regions (32) which are remote from the front region (30) the mounting plate (18) has at least one respective blind hole (33, 34), which holes extend in mutually parallel relationship and in a plane in which the mounting plate (18) extends, securing pins which project beyond the rim portion (28) in a direction towards the blind holes (33, 34) extending into the holes.

**Revendications**

**1.** Endoprothèse d'une articulation, consistant en deux composants (1, 2) non reliés l'un à l'autre, qui sont montés avec possibilité de translation l'un par rapport à l'autre dans la direction du flux des forces existant entre eux, caractérisée par le fait qu'entre les composants (1, 2), est disposée au moins une butée (11) limitant l'étendue de la possibilité de translation des composants (1, 2) l'un par rapport à l'autre, qui est réalisée sous la forme d'une collerette (12) entourant radialement au moins partiellement la pièce de guidage (10), qui est en prise avec un rebord de butée (13) prévu sur l'autre composant (2).

**2.** Endoprothèse selon la revendication 1, caractérisée par le fait qu'elle est réalisée sous la forme d'une endoprothèse de l'articulation du genou qui comporte un composant fémoral et un composant tibial (1, 2).

**3.** Endoprothèse selon l'une quelconque des revendications 1 et 2, caractérisée par le fait qu'entre les composants (1, 2), est disposé au moins à chaque fois un support (7, 8) autorisant des mouvements de fléchissement autour de leurs axes transversaux et des mouvements de rotation autour de leurs axes longitudinaux respectivement l'un par rapport à l'autre.

**4.** Endoprothèse selon l'une quelconque des revendications 1 à 3, caractérisée par le fait qu'un support pivotant autorisant les mouvements de fléchissement est réalisé sous la forme d'une charnière qui est fixée sur l'un (1) des composants et qui présente une pièce de guidage (10), laquelle est réalisée sous la forme d'un axe de charnière (9) et est montée déplaçable en rotation et coulissante sur l'autre composant (2).

**5.** Endoprothèse selon l'une quelconque des revendications 2 à 4, caractérisée par le fait la collerette (12) est prévue sur le composant fémoral (1) et le rebord de butée (13), sur le composant tibial (2).

**6.** Endoprothèse selon l'une quelconque des revendications 4 et 5, caractérisée par le fait la charnière est fixée sur le composant fémoral (1) et que dans la pièce de guidage (10), pénètre un pivot (14) autorisant ses mouvements de rotation et ses mouvements de coulissement, lequel est rendu solidaire du composant tibial (2), et que la collerette (12) est disposée sur une extrémité (15) de la pièce de guidage (10), qui est tournée vers le composant tibial (2).

**7.** Endoprothèse selon la revendication 6, caractérisée par le fait que la collerette (12) dépasse au moins partiellement de la pièce de guidage (10) dans des directions qui sont opposées au pivot (14).

**8.** Endoprothèse selon la revendication 7, caractérisée par le fait que la collerette (12) dépasse de la pièce de guidage (10) sous la forme d'une bride annulaire qui présente, sur son côté opposé au composant tibial (2), une surface d'appui (17), sur laquelle vient en appui le rebord de butée (13) lorsqu'il atteint une position finale présélectionnée dans laquelle les composants (1, 2) sont le plus écartés l'un de l'autre.

9. Endoprothèse selon l'une quelconque des revendications 3 à 8, caractérisée par le fait que la collerette (12) est aplatie dans une zone arrière (16) de la pièce de guidage, dans laquelle les deux composants (1, 2) se rapprochent le plus lors de l'exécution des mouvements de fléchissement.

10. Endoprothèse selon l'une quelconque des revendications 2 à 9, caractérisée par le fait que le rebord de butée (13) est prévu sur une moitié de support tibiale (5), qui délimite le composant tibial (2) dans la direction du composant fémoral (1).

11. Endoprothèse selon la revendication 10, caractérisée par le fait que la moitié de support tibiale (5) se compose d'une plaque-support (18) sensiblement en forme de fer à cheval, qui entoure partiellement le pivot (14) qui s'étend sensiblement transversalement par rapport à la direction longitudinale du composant tibial (2) et qui est dotée, sur sa surface (19) tournée du côté du composant fémoral (1), de surfaces de glissement (20) sur lesquelles s'appuie une moitié de support fémoral.

12. Endoprothèse selon la revendication 11, caractérisée par le fait que la plaque-support (18) présente une cavité (21), sensiblement en forme de U, qui la traverse sensiblement dans la direction longitudinale du composant tibial (2) et qui présente une zone (22) arrondie avant, qui est tournée vers une zone avant (23) de la collerette (12), qui est en prise avec le rebord de butée (13).

13. Endoprothèse selon la revendication 12, caractérisée par le fait que la cavité (21) est ajustée, sur sa zone arrondie (22), à une partie (24) tournée vers elle de la collerette (12).

14. Endoprothèse selon l'une quelconque des revendications 12 et 13, caractérisée par le fait que le rebord de butée (13) pénètre dans la zone arrondie (22) de la cavité (21) dans la direction du pivot (14).

15. Endoprothèse selon l'une quelconque des revendications 12 à 14, caractérisée par le fait que le rebord de butée (13) limite la zone arrondie (22) dans la direction du composant fémoral (1).

16. Endoprothèse selon l'une quelconque des revendications 12 à 15, caractérisée par le fait que le rebord de butée (13) est plié et s'étend sensiblement concentriquement à la zone arrondie (22).

17. Endoprothèse selon l'une quelconque des revendications 11 à 16, caractérisée par le fait que le rebord de butée (13) respecte une distance par rapport à une surface (25) d'un support (26) portant la plaque-support (18) du composant tibial (2).

18. Endoprothèse selon la revendication 17, caractérisée par le fait qu'entre le rebord de butée (13) et la surface (25), est prévu un espace libre (27), qui présente la hauteur de la distance entre le rebord de butée (13) et la surface (25) et que la collerette (12) est montée à translation et à rotation dans l'espace libre (27).

19. Endoprothèse selon l'une quelconque des revendications 12 à 18, caractérisée par le fait que la collerette (12) présente un jeu par rapport à la cavité (21).

20. Endoprothèse selon l'une quelconque des revendications 17 à 19, caractérisée par le fait que la plaque-support (18) est fixée sur le support (26).

21. Endoprothèse selon l'une quelconque des revendications 17 à 20, caractérisée par le fait que le support (26) est entouré, sur sa surface (25), par un rebord (28) sur lequel est adaptée la plaque-support (18) avec sa surface inférieure (29) tournée vers le support (26).

22. Endoprothèse selon l'une quelconque des revendications 17 à 21, caractérisée par le fait que la plaque-support (18) est ajustée géométriquement à la surface (25).

23. Endoprothèse selon l'une quelconque des revendications 20 à 22, caractérisée par le fait que la plaque-support (18) est fixée, dans sa zone avant (30), sur le support (26), par une vis (31) qui est enfoncée au moins partiellement dans la plaque-support (18).

24. Endoprothèse selon l'une quelconque des revendications 20 à 23, caractérisée par le fait que la plaque-support (18) présente, sur ses zones (32) tournées à l'opposé de la zone avant (30), au moins à chaque fois un trou borgne (33, 34), qui s'étendent parallèlement l'un à l'autre et dans un plan s'étendant à partir de la plaque support (18) et dans lesquels pénètrent des goupilles de sécurité qui dépassent du rebord (28) dans la direction des trous borgnes (33, 34).

Fig. 2

32    21    32

13    22    29    18

Fig. 3

30

13    18    34

32    32

29    33    21    V    Fig. 4

V

13    18

30    32

34

29    Fig. 5

VII

9

10

17    12

24    24

VII    Fig. 6

9

12    10

17    16

23    15    Fig. 7

IX

33    34

Fig. 8    IX

13    12

30    32

Fig. 9

11